# EUROPÄISCHE PATENTSCHRIFT

(11) **EP 0 287 926 B1**
(45) Veröffentlichungstag und Bekanntmachung des Hinweises auf die Patenterteilung: **01.12.1993**
(21) Anmeldenummer: 88105766.5
(22) Anmeldetag: 12.04.1988
(51) Int. Cl.: A61K 31/47, C07D 215/56

(54) **Verfahren zur Herstellung von parenteral verabreichbaren Chinoloncarbonsäuren**
Process for the preparation of quinoline carboxylic acids for parenteral application
Procédé de préparation d'acides quinoléinecarboxyliques pour application parentérale

(30) Priorität: 24.04.1987 DE 3713672
(43) Veröffentlichungstag der Anmeldung: 26.10.1988
(73) Patentinhaber: BAYER AG, 51368 Leverkusen (DE)
(72) Erfinder: Preiss, Michael, Dr., D-5600 Wuppertal 1 (DE); Schorsch, Ulrich, Dr., D-4000 Düsseldorf 12 (DE); Haaf, Arthur, Dr., D-5600 Wuppertal 21 (DE); Naab, Paul, Dr., D-5600 Wuppertal 21 (DE); Zerbes, Rudolf, Dr., D-5600 Wuppertal 1 (DE)

(56) Entgegenhaltungen:
- FR-A- 2 498 931

## Beschreibung

Die Erfindung betrifft ein neues Verfahren zur Herstellung von Chinoloncarbonsäuren, die zur Herstellung von parenteral verabreichbaren Lösungen geeignet sind.

Bei der Synthese fallen bereits sehr reine Chinoloncarbonsäuren an. Diese sind jedoch zur Herstellung von Injektions- und Infusionslösungen nicht geeignet, da letztere nicht lagerstabil sind. Nach einiger Zeit treten Ausscheidungen auf, wodurch die Lösungen unbrauchbar werden.

Nach allen bisher untersuchten Verfahren, sei es das in FR-A-24 98 931 beschriebene, oder z.B. durch Umfällen und Umkristallisieren, gelang es nicht, Chinoloncarbonsäuren zu erhalten, aus denen lagerstabile Lösungen hergestellt werden können.

Ein Gegenstand der Erfindung sind parenteral verabreichbare Lösungen von Chinoloncarbonsäuren, die dadurch gekennzeichnet sind, daß sie Chinoloncarbonsäuren bzw. deren Derivate der allgemeinen Formel (I) 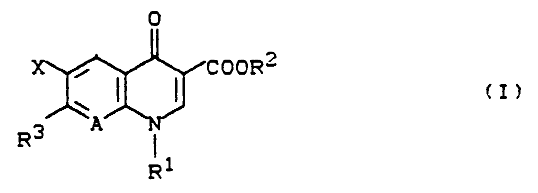
in welcher
- R¹: für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorophenyl,
- R²: für Wasserstoff, Alkyl mit 1 bis 4 Kohlenstoffatomen, (5-Methyl-2-oxo-1,3-dioxol-4-yl)-methyl,
- R³: für Methyl oder eine cyclische Aminogruppe wie 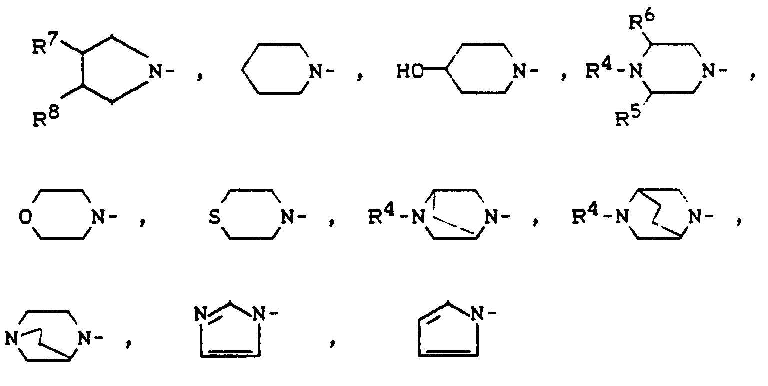
steht, worin
- R⁴: für Wasserstoff, Alkyl mit 1 bis 4 Kohlenstoffatomen, 2-Hydroxyethyl, Allyl, Propargyl, 2-Oxo-propyl, 3-Oxobutyl, Phenacyl, Formyl. CFCl₂-S-, CFCl₂-SO₂-, CH₃O-CO-S-, Benzyl, 4-Aminobenzyl, 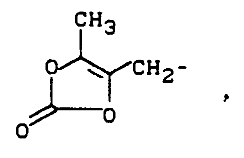
- R⁵: für Wasserstoff, Methyl,
- R⁶: für Wasserstoff, Alkyl mit 1 bis 4 Kohlenstoffatomen, Phenyl, Benzyloxymethyl,
- R⁷: für Wasserstoff, Amino, Methylamino, Ethylamino, Aminomethyl, Methylaminomethyl, Ethylaminomethyl, Dimethylaminomethyl, Hydroxy, Hydroxymethyl,
- R⁸: für Wasserstoff, Methyl, Ethyl oder Chlor steht,
- X: für Fluor, Chlor oder Nitro und
- A: für N oder C-R⁹ steht, worin
R⁹ für Wasserstoff, Halogen wie Fluor oder Chlor, Methyl oder Nitro steht oder auch gemeinsam mit R¹ eine Brücke der Struktur 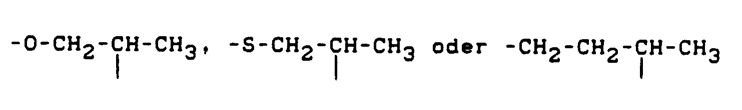 bilden kann,
in gelöster Form mit nicht mehr als 1 bis 10 ppm, bevorzugt 1 bis 5 ppm, bezogen auf die Hauptwirkstoffkomponente der Lösung, an Nebenkomponenten enthalten.

Gegenstand der Erfindung ist ein Verfahren, in dem bereits sehr reine Chinoloncarbonsäuren einer speziellen Behandlung unterzogen werden, so daß daraus lagerstabile Lösungen gefertigt werden können.

Insbesondere betrifft die Erfindung ein Verfahren zur Herstellung parenteral verabreichbarer Lösungen von Chinoloncarbonsäuren, die Chinoloncarbonsäuren bzw. deren Derivate der allgemeinen Formel (I) 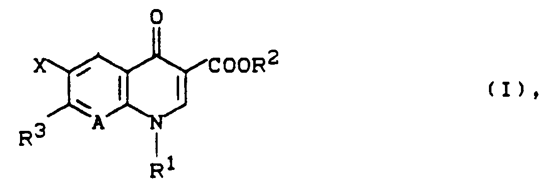
in welcher
- R¹: für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
- R²: für Wasserstoff, Alkyl mit 1 bis 4 Kohlenstoffatomen, (5-Methyl-2-oxo-1,3-dioxol-4-yl)-methyl,
- R³: für Methyl oder eine cyclische Aminogruppe wie 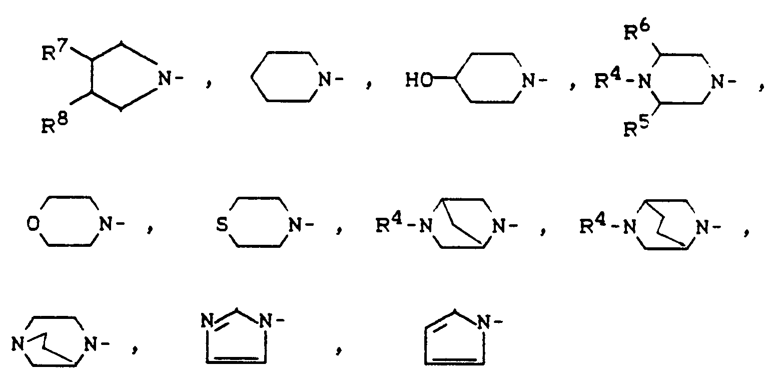 steht, worin
- R⁴: für Wasserstoff, Alkyl mit 1 bis 4 Kohlenstoffatomen, 2-Hydroxyethyl, Allyl, Propargyl, 2-Oxo-propyl, 3-Oxobutyl, Phenacyl, Formyl, CFCl₂-S-, CFCl₂-SO₂-, CH₃O-CO-S-, Benzyl, 4-Aminobenzyl, 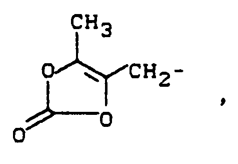
- R⁵: für Wasserstoff, Methyl,
- R⁶: für Wasserstoff, Alkyl mit 1 bis 4 Kohlenstoffatomen, Phenyl, Benzyloxymethyl,
- R⁷: für Wasserstoff, Amino, Methylamino, Ethylamino, Aminomethyl, Methylaminomethyl, Ethylaminomethyl, Dimethylaminomethyl, Hydroxy, Hydroxymethyl,
- R⁸: für Wasserstoff, Methyl, Ethyl oder Chlor steht,
- X: für Fluor, Chlor oder Nitro und
- A: für N oder C-R⁹ steht, worin
R⁹ für Wasserstoff, Halogen wie Fluor oder Chlor, Methyl oder Nitro steht oder auch gemeinsam mit R¹ eine Brücke der Struktur 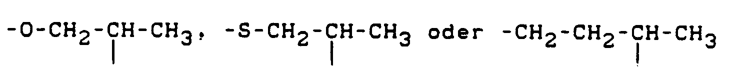 bilden kann,
in gelöster Form mit nicht mehr als 1 bis 10 ppm, bevorzugt 1 bis 5 ppm, bezogen auf die Hauptwirkstoffkomponente der Lösung, an Nebenkomponenten enthalten,
dadurch gekennzeichnet, daß man einen Wirkstoff der Formel I mit Säure löst, die Lösung bei Temperaturen von Raumtemperatur bis zum Siedepunkt der Lösung, gegebenenfalls in Gegenwart von Aktivkohle, während 0,15 bis 150 Stunden, vorzugsweise 0,25 bis 110 Stunden, gegebenenfalls unter Rühren stehen läßt, die Lösung filtriert, die Chinoloncarbonsäuren mit basischen Reagentien ausfällt und die ausgefällte Chinoloncarbonsäure mit den üblichen Hilfsmitteln in eine parenteral applizierbare Darreichungsform bringt.

Bevorzugt ist das erfindungsgemäße Verfahren der Form, daß man als Wirkstoffe solche der allgemeinen Formel (I) verwendet, in denen R³ für die Reste 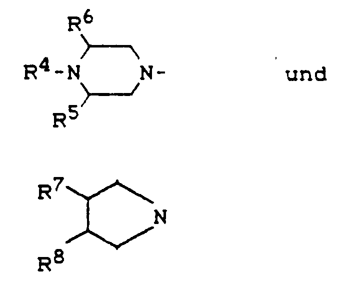
steht,
worin R⁴, R⁵, R⁶, R⁷ und R⁸ die oben angegebene Bedeutung haben.

Besonders bevorzugt wird das erfindungsgemäße Verfahren so durchgeführt, daß man als Wirkstoffe solche der allgemeinen Formel I verwendet, in denen X für Fluor oder Chlor und A für CH, C-Cl, C-F oder C-Br steht, oder daß man als Wirkstoffe solche der allgemeinen Formel I verwendet, in denen X für Fluor und A für CH, C-Cl oder CF steht und R³ den Rest 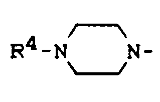
mit R₄ = H, C₂H₅, CH₃ oder -CH₂-CH₂-OH oder die Reste 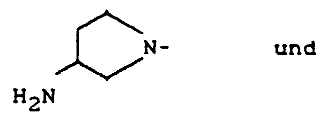 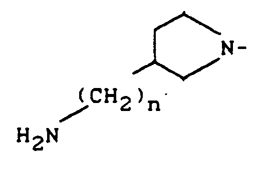
mit n = 1 bis 4 bedeutet.

Das Verfahren umfaßt das Aufbewahren von bei der jeweiligen Temperatur gesättigten salzsauren Lösungen der Verbindungen der Formel I bei Raumtemperatur bis zum Siedepunkt der Lösung in Gegenwart von Aktivkohle oder auch ohne und Standzeiten von 0,15 bis 150, bevorzugt von 0,25 bis 110 Stunden. Als Filterhilfsmittel sind z.B. die verschiedensten Kieselgurarten geeignet. Desgleichen können die verschiedensten Kohlearten eingesetzt werden. Die zum Ausfällen verwendeten basischen Mittel können Alkali- und Erdalkalihydroxide, Ammoniak oder organische Basen wie z.B. tertiäre Amine sein.

Das erfindungsgemäße Verfahren wird beispielsweise so ausgeführt, daß die Chinoloncarbonsäure selbst oder in Form ihrer Salze, gegebenenfalls unter Säurezusatz, in Wasser oder anderen geeigneten Lösungsmitteln bei Temperaturen von Raumtemperatur bis zum Siedepunkt des Lösungsmittels bis zur Sättigung gelöst wird. Nach dem Lösen schließen sich die übrigen Maßnahmen an.

Als Säuren kommen infrage: eine zur Lösung des Wirkstoffs ausreichende Menge einer oder mehrerer Säure(n) im Gemisch mit Wasser aus der Gruppe bestehend aus Salzsäure, Methansulfonsäure, Propionsäure, Bernsteinsäure, Glutarsäure, Zitronensäure, Fumarsäure, Maleinsäure, Weinsäure, Glutaminsäure, Glukonsäure, Glukuronsäure, Galakturonsäure, Ascorbinsäure, Phosphorsäure, Adipinsäure, Hydroxyessigsäure, Schwefelsäure, Salpetersäure, Essigsäure, Apfelsäure, L-Asparaginsäure und Milchsäure.

Milchsäure und Salzsäure bzw. Gemische von Milchsäure und Salzsäure sind besonders bevorzugt.

Die Mengen sind durch einfache Handversuche zu ermitteln.

Die erfindungsgemäß gereinigten Wirkstoffe können direkt zugesetzt werden oder nach Lehre der EP-A-13 80 18-A3 und EPA-86 11 41 31.5 zu Infusionslösungen etc. verarbeitet werden.

Ganz besonders bevorzugt wird in dem erfindungsgemäßen Verfahren Ciprofloxacin als Wirkstoff verwendet.

Zweckmäßigerweise läßt man bei der jeweiligen Temperatur gesättigte salzsaure Lösungen der Ciprofloxacin 0,15 bis 150 Stunden stehen, wobei Aktivkohle eingesetzt wird.

Weiterer Gegenstand der Erfindung sind Lösungen bzw. Wirkstoffe, die nach den anspruchsgemäßen Verfahren hergestellt werden, zur Anwendung in einem Verfahren zur therapeutischen Behandlung des menschlichen oder tierischen Körpers.

Es ist als ausgesprochen unerwartet zu bezeichnen, daß durch Aufbewahren der salzsauren Lösungen der Chinoloncarbonsäuren der Formel Iʹ 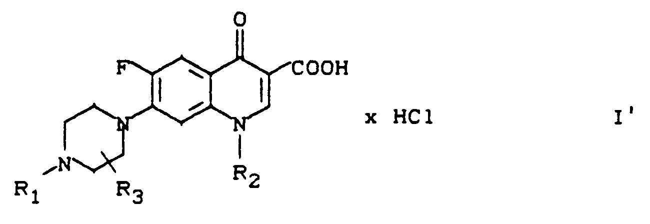
über einen bestimmten Zeitraum in Gegenwart von Aktivkohle oder auch ohne,Filtration der salzsauren Lösungen mit oder ohne Filterhilfsmittel und anschließendem Fällen mit basischen Mitteln, Chinoloncarbonsäuren erhalten werden, deren Lösungen lagerstabil sind. Die Chinoloncarbonsäuren liegen als Betaine vor, deren Struktur wie in Formel II 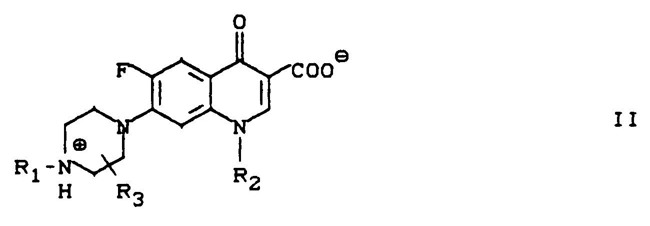
dargestellt wiedergegeben werden kann.

Die Ausscheidungen sind wahrscheinlich auf schwerlösliche Polykondensationsprodukte, die eine sehr geringe Kristallwachstumsrate besitzen, zurückzuführen. Bedingt durch die geringe Kristallwachstumsrate geben solche Verbindungen Anlaß zu übersättigten Lösungen, die nach unbestimmter Zeit ausflocken.

So konnten beispielsweise in Lösungen von Verbindung III 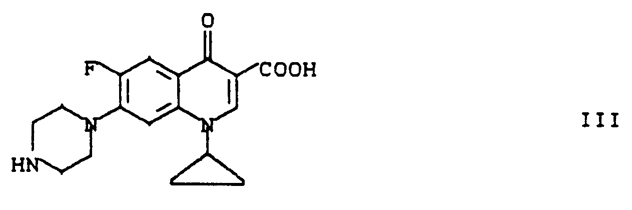
Verbindungen der allgemeinen Formel IV 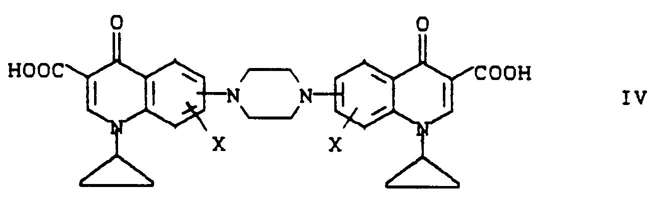
im ppm-Bereich nachgewiesen werden.

Durch das beschriebene Verfahren gelingt es, die Mengen der Verbindungen der Formel IV, von denen beispielsweise Verbindung VI genannt sei, derart zu 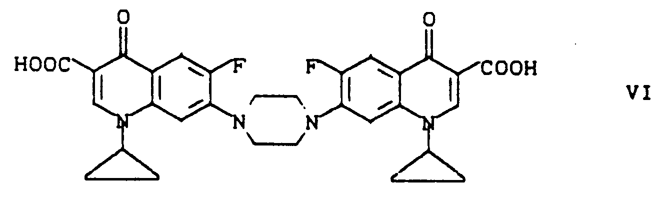
vermindern, daß lagerstabile Lösungen von Verbindungen III erhalten werden.

Andere Verfahren zur Entfernung dieser außerordentlich geringen Anteile von Verbindungen der allgemeinen Formel IV führten nicht zum Ziel. So gelang es nicht, durch Umkristallisation von Verbindungen der Formel I oder entsprechender Salze obige Nebenprodukte zu entfernen. Umfällen der Verbindungen der Formel I in alkalischem Medium brachte ebenfalls kein brauchbares Ergebnis, desgleichen Zusatz von Lösungsmitteln der verschiedensten Art.

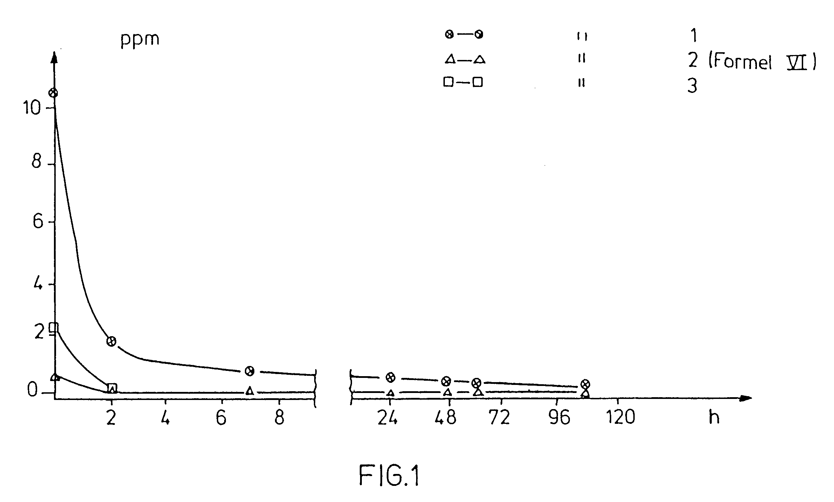

Fig. 1 zeigt partikelrelevante Nebenprodukte für den Wirkstoff Ciprofloxacin. Aufgeführt sind hier beispielsweise die Nebenprodukte 1, 2, und 3. Die Struktur von Nebenprodukt 2 ist in Formel VI wiedergegeben.

### Beispiel 1

30 Gew.-Teile Ciprofloxacin-Hydrochlorid und 3 Gew.-Teile Aktivkohle werden in 1080 Gew.-Teilen Wasser 2 Stunden gerührt. Danach wird filtriert und mit Kalilauge auf pH 7,8 gestellt. Der Niederschlag wird abgetrennt, gewaschen und getrocknet. Ausbeute: 22 Gew.-Teile Ciprofloxacin.

### Beispiel 2

Wie Beispiel 1; die Reaktionszeit beträgt jedoch 48 Stunden.

### Beispiel 3

Wie Beispiel 1; die Reaktionszeit beträgt jedoch 108 Stunden.

### Beispiel 4

Wie Beispiel 1; statt Kalilauge wird Natronlauge eingesetzt.

### Beispiel 5

Wie Beispiel 2; statt Kalilauge wird Ammoniakwasser eingesetzt.

### Beispiel 6

Wie Beispiel 3; statt Kalilauge wird Triethylamin eingesetzt.

### Beispiel 7

41 Gew.-Teile Ciprofloxacin-Hydrochlorid und 4,1 Gew.-Teile Aktivkohle werden in 700 Gew.-Teilen Wasser 60 Stunden bei 30°C gerührt. Die Aufarbeitung erfolgt wie in Beispiel 1. Ausbeute: 31,6 Gew.-Teile Ciprofloxacin.

### Beispiel 8

82 Gew.-Teile Ciprofloxacin-Hydrochlorid und 8,2 Gew.-Teile Aktivkohle werden in 700 Gew.-Teilen Wasser 60 Stunden bei 50° C gerührt. Die Aufarbeitung erfolgt wie in Beispiel 1. Ausbeute: 65,7 Gew.-Teile Ciprofloxacin.

### Beispiel 9

Wie Beispiel 1; statt Aktivkohle werden 3 Gew.-Teile Kieselgur eingesetzt.

### Beispiel 10

Wie Beispiel 1; es werden 1,5 Gew.-Teile Aktivkohle und 1,5 Gew.-Teile Kieselgur eingesetzt.

### Beispiel 11

Wie Beispiel 1; jedoch ohne Aktivkohle.

### Beispiel 12

Wie Beispiel 8; jedoch ohne Aktivkohle.

## Patentansprüche (Patentansprüche für folgende(n) Vertragsstaat(en): AT, BE, CH, DE, FR, GB, IT, LI, NL, SE)

1. Lagerstabile, parenteral verabreichbare Lösungen von Chinoloncarbonsäuren, dadurch gekennzeichnet, daß sie Chinoloncarbonsäuren bzw. deren Derivate der allgemeinen Formel (I) 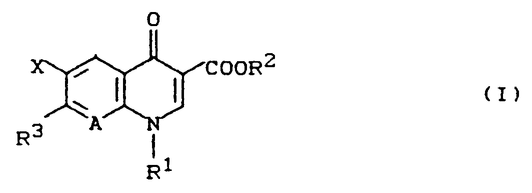 in welcher
R¹ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
R² für Wasserstoff, Alkyl mit 1 bis 4 Kohlenstoffatomen. (5-Methyl-2-oxo-1,3dioxol-4-yl)-methyl,
R³ für Methyl oder eine cyclische Aminogruppe wie 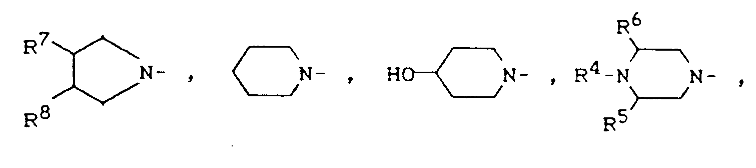 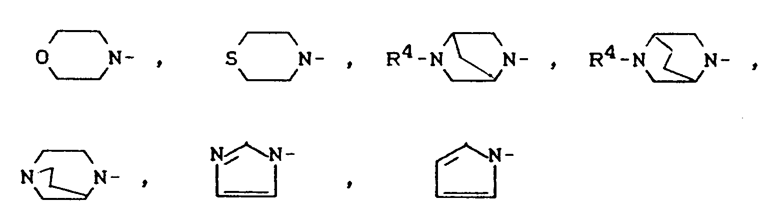 steht, worin
R⁴ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, CFCl₂-S-, CFCl₂-SO₂-, CH₃O-CO-S-, Benzyl, 4-Aminobenzyl, 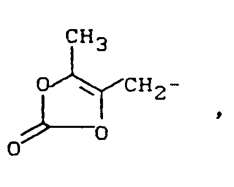
R⁵ für Wasserstoff, Methyl,
R⁶ für Wasserstoff, Alkyl mit 1 bis 4 Kohlenstoffatomen, Phenyl, Benzyloxymethyl,
R⁷ für Wasserstoff, Amino, Methylamino, Ethylamino, Aminomethyl, Methylaminomethyl, Ethylaminomethyl, Dimethylaminomethyl, Hydroxy, Hydroxymethyl,
R⁸ für Wasserstoff, Methyl, Ethyl oder Chlor steht,
X für Fluor, Chlor oder Nitro und
A für N oder C-R⁹ steht, worin
R⁹ für Wasserstoff, Halogen wie Fluor oder Chlor, Methyl oder Nitro steht oder auch gemeinsam mit R¹ eine Brücke der Struktur 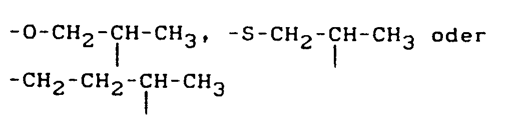 bilden kann,
in gelöster Form mit nicht mehr als 1 bis 10 ppm, bevorzugt 1 bis 5 ppm, bezogen auf die Hauptwirkstoffkomponente der Lösung, an insbesondere nach Lagerung der Lösung zur Bildung von Ausfällungen neigenden Nebenkomponenten enthalten.

2. Verfahren zur Herstellung parenteral verabreichbarer Lösungen gemäß Anspruch 1,
dadurch gekennzeichnet, daß man einen Wirkstoff der Formel I mit Säure löst, die Lösung bei Temperaturen von Raumtemperatur bis zum Siedepunkt der Lösung, gegebenenfalls in Gegenwart von Aktivkohle, während 0,15 bis 150 Stunden, vorzugsweise 0,25 bis 110 Stunden, gegebenenfalls unter Rühren stehen läßt, die Lösung filtriert, die Chinoloncarbonsäure mit basischen Reagentien ausfällt und die ausgefällte Chinoloncarbonsäure mit den üblichen Hilfsmitteln in eine parenteral applizierbare Darreichungsform bringt.

3. Verfahren nach Anspruch 2, dadurch gekennzeichnet, daß man als Wirkstoffe solche der allgemeinen Formel (I) verwendet, in denen R³ für die Reste 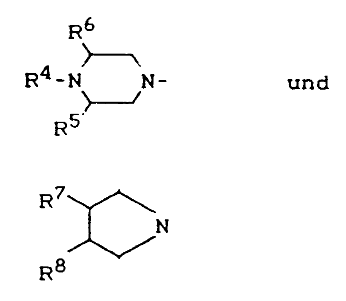 steht,
worin R⁴, R⁵, R⁶, R⁷ und R⁸ die in Anspruch 1 angegebene Bedeutung haben.

4. Verfahren nach Anspruch 3, dadurch gekennzeichnet, daß man als Wirkstoffe solche der allgemeinen Formel I verwendet, in denen X für Fluor oder Chlor und A für CH, C-Cl, C-F oder C-Br steht.

5. Verfahren nach Anspruch 4, dadurch gekennzeichnet, daß man als Wirkstoffe solche der allgemeinen Formel I verwendet, in denen X für Fluor und A für CH, C-Cl oder CF steht und R³ den Rest 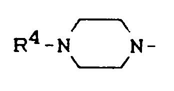 mit R₄ = H, C₂H₅, CH₃ oder -CH₂-CH₂-OH oder die Reste 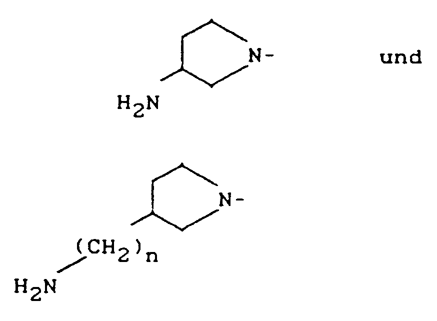 mit n = 1 bis 4 bedeutet.

6. Verfahren nach Anspruch 2, dadurch gekennzeichnet, daß man Ciprofloxacin als Wirkstoff verwendet.

7. Verfahren nach Anspruch 6, dadurch gekennzeichnet, daß man bei der jeweiligen Temperatur gesättigte salzsaure Lösungen des Ciprofloxacin 0,15 bis 150 Stunden stehen läßt und Aktivkohle einsetzt.

8. Verfahren nach Anspruch 6, dadurch gekennzeichnet, daß man zur Ausfällung Alkali- und Erdalkalihydroxide, Ammoniak oder organische Basen verwendet.

9. Lösungen bzw. Wirkstoffe, die nach dem Verfahren des Anspruchs 2 hergestellt werden, zur Anwendung in einem Verfahren zur therapeutischen Behandlung des menschlichen oder tierischen Körpers.

## Patentansprüche (Patentansprüche für folgende(n) Vertragsstaat(en): GR, ES)

1. Verfahren zur Herstellung lagerstabiler, parenteral verabreichbarer Lösungen, enthaltend Chinoloncarbonsäuren bzw. deren Derivate der allgemeinen Formel (I) 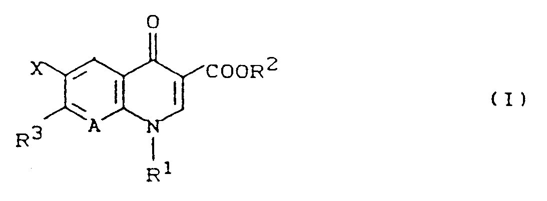 in welcher
R¹ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
R² für Wasserstoff, Alkyl mit 1 bis 4 Kohlenstoffatomen, (5-Methyl-2-oxo-1,3-dioxol-4-yl)-methyl,
R³ für Methyl oder eine cyclische Aminogruppe wie 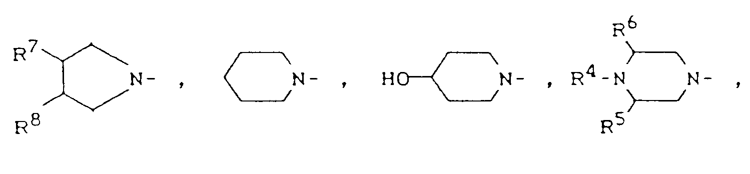 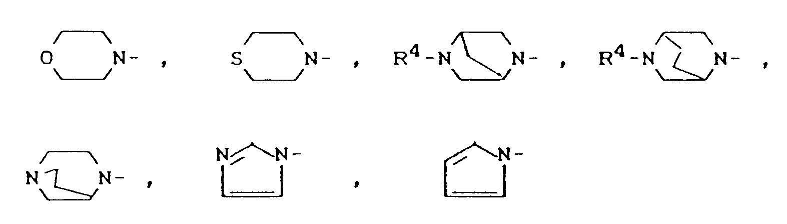 steht, worin
R⁴ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, CFCl₂-S-, CFCl₂-SO₂-, CH₃O-CO-S-, Benzyl, 4-Aminobenzyl, 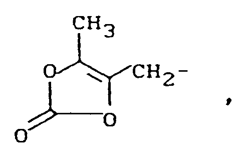
R⁵ für Wasserstoff, Methyl,
R⁶ für Wasserstoff, Alkyl mit 1 bis 4 Kohlenstoffatomen, Phenyl, Benzyloxymethyl,
R⁷ für Wasserstoff, Amino, Methylamino, Ethylamino, Aminomethyl, Methylaminomethyl, Ethylaminomethyl, Dimethylaminomethyl, Hydroxy, Hydroxymethyl,
R⁸ für Wasserstoff, Methyl, Ethyl oder Chlor steht,
X für Fluor, Chlor oder Nitro und
A für N oder C-R⁹ steht, worin R⁹ für Wasserstoff, Halogen wie Fluor oder Chlor, Methyl oder Nitro steht oder auch gemeinsam mit R¹ eine Brücke der Struktur 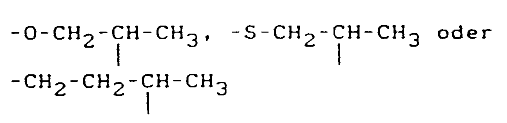 bilden kann,
in gelöster Form mit nicht mehr als 1 bis 10 ppm, bevorzugt 1 bis 5 ppm, bezogen auf die Hauptwirkstoffkomponente der Lösung, an insbesondere nach Lagerung der Lösung zur Bildung von Ausfällungen neigenden Nebenkomponenten enthalten, dadurch gekennzeichnet, daß man einen Wirkstoff der Formel (I) mit Säure löst, die Lösung bei Temperaturen von Raumtemperatur bis zum Siedepunkt der Lösung, gegebenenfalls in Gegenwart von Aktivkohle, während 0,15 bis 150 Stunden, vorzugsweise 0,25 bis 110 Stunden, gegebenenfalls unter Rühren stehen läßt, die Lösung filtriert, die Chinoloncarbonsäure mit basischen Reagentien ausfällt und die ausgefällte Chinoloncarbonsäure mit den üblichen Hilfsmitteln in eine parenteral applizierbare Darreichungsform bringt.

2. Verfahren nach Anspruch 1, dadurch gekennzeichnet, daß man als Wirkstoffe solche der allgemeinen Formel (I) verwendet, in denen R³ für die Reste 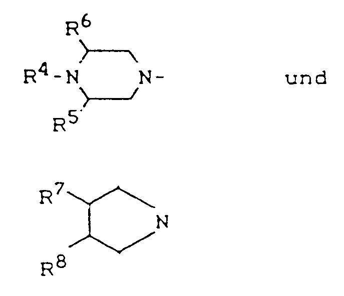 steht,
worin R⁴, R⁵, R⁶, R⁷ und R⁸ die in Anspruch 1 angegebene Bedeutung haben.

3. Verfahren nach Ansprüchen 1 und 2, dadurch gekennzeichnet, daß man als Wirkstoffe solche der allgemeinen Formel (I) verwendet, in denen X für Fluor oder Chlor und A für CH, C-Cl, C-F oder C-Br steht.

4. Verfahren nach Anspruch 1, dadurch gekennzeichnet, daß man als Wirkstoffe solche der allgemeinen Formel (I) verwendet, in denen X für Fluor und A für CH, C-Cl oder CF steht und R³ den Rest 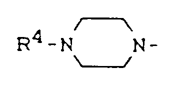 mit R₄ = H, C₂H₅, CH₃ oder -CH₂-CH₂-OH oder die Reste 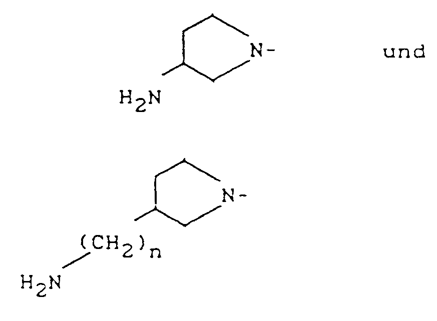 mit n = 1 bis 4 bedeutet.

5. Verfahren nach Anspruch 1, dadurch gekennzeichnet, daß man Ciprofloxacin als Wirkstoff verwendet.

6. Verfahren nach Anspruch 5, dadurch gekennzeichnet, daß man bei der jeweiligen Temperatur gesättigte salzsaure Lösungen des Ciprofloxacin 0,15 bis 150 Stunden stehen läßt und Aktivkohle einsetzt.

7. Verfahren nach Anspruch 6, dadurch gekennzeichnet, daß man zur Ausfällung Alkali und Erdalkalihydroxide, Ammoniak oder organische Basen verwendet.

## Claims (Claims for the following Contracting State(s): AT, BE, CH, DE, FR, GB, IT, LI, NL, SE)

1. Quinolonecarboxylic acid solutions which have a long shelf life and can be administered parenterally, characterised in that they contain quinolonecarboxylic acids, or derivatives thereof, of the general formula (I) 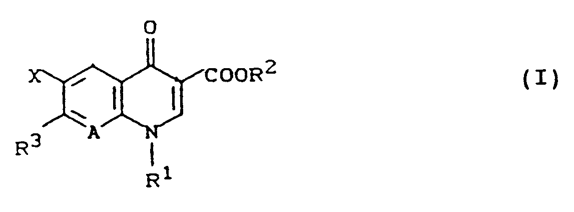 in which
R¹ represents methyl, ethyl, propyl, isopropyl, cyclopropyl, vinyl, 2-hydroxyethyl, 2-fluoroethyl, methoxy, amino, methylamino, dimethylamino, ethylamino, phenyl, 4-fluorophenyl or 2,4-difluorophenyl,
R² represents hydrogen, alkyl having 1 to 4 carbon atoms or (5-methyl-2-oxo-1,3-dioxol-4-yl)-methyl,
R³ represents methyl or a cyclic amino group, such as 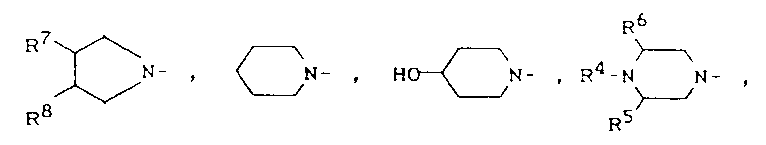 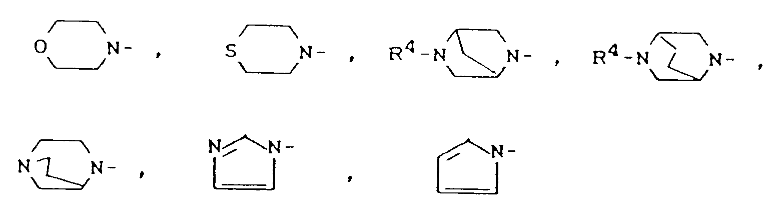 in which
R⁴ represents hydrogen, alkyl having 1 to 4 carbon atoms, 2-hydroxyethyl, allyl, propargyl, 2-oxopropyl, 3-oxobutyl, phenacyl, formyl, CFCl₂-S-CFCl₂-SO₂-, CH₃O-CO-S-, benzyl, 4-aminobenzyl 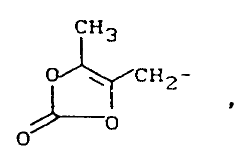
R⁵ represents hydrogen or methyl,
R⁶ represents hydrogen, alkyl having 1 to 4 carbon atoms, phenyl or benzyloxymethyl,
R⁷ represents hydrogen, amino, methylamino, ethylamino, aminomethyl, methylaminomethyl, ethylaminomethyl, dimethylaminomethyl, hydroxyl or hydroxymethyl,
R⁸ represents hydrogen, methyl, ethyl or chlorine,
X represents fluorine, chlorine or nitro, and
A represents N or C-R⁹, in which
R⁹ represents hydrogen, halogen, such as fluorine or chlorine, methyl or nitro, or alternatively, together with R¹, can form a bridge of the structure 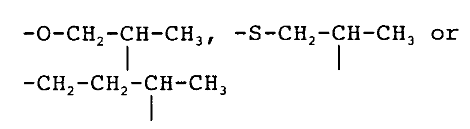
in dissolved form with no more than 1 to 10 ppm, preferably 1 to 5 ppm, relative to the major active compound component of the solution, of secondary components which have a tendency to precipitate, in particular after storage of the solution.

2. Process for the production of solutions according to Claim 1 which can be administered parenterally, characterised in that an active compound of the formula I is dissolved using acid, the solution is allowed to stand, if appropriate with stirring, for 0.15 to 150 hours, preferably 0.25 to 110 hours, at temperatures from room temperature to the boiling point of the solution, if appropriate in the presence of activated charcoal, the solution is filtered, the quinolonecarboxylic acid is precipitated using basic reagents, and the precipitated quinolonecarboxylic acid is converted, using conventional auxiliaries, into an administration form which can be administered parenterally.

3. Process according to Claim 2, characterised in that the active compounds employed are those of the general formula (I) in which R³ represents the 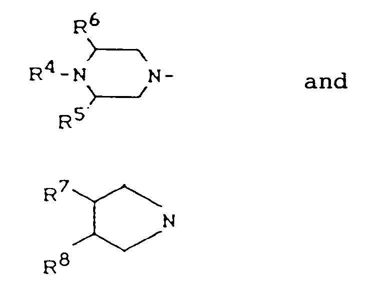 radicals,
in which R⁴, R⁵, R⁶, R⁷ and R⁸ have the meaning specified in Claim 1.

4. Process according to Claim 3, characterised in that the active compounds employed are those of the general formula I in which X represents fluorine or chlorine and A represents CH, C-Cl, C-F or C-Br.

5. Process according to Claim 4, characterised in that the active compounds employed are those of the general formula I in which X represents fluorine and A represents CH, C-Cl or CF, and R³ denotes the 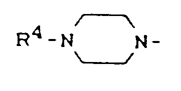 radical where R₄ = H, C₂H₅, CH₃ or -CH₂-CH₂-OH or the 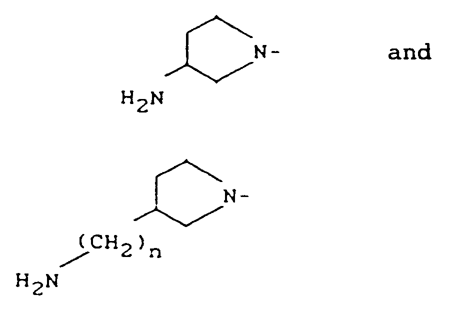 radicals where n = 1 to 4.

6. Process according to Claim 2, characterised in that the active compound used is ciprofloxacin.

7. Process according to Claim 6, characterised in that saturated ciprofloxacin solutions containing hydrochloric acid are allowed to stand for 0.15 to 150 hours at the particular temperature, and activated charcoal is employed.

8. Process according to Claim 6, characterised in that alkali metal hydroxides, alkaline-earth metal hydroxides, ammonia or organic bases are used for the precipitation.

9. Solutions or active compounds which are prepared by the process of Claim 2, for use in a process for therapeutic treatment of the human or animal body.

## Claims (Claims for the following Contracting State(s): GR, ES)

1. Process for the production of solutions which have a long shelf life and can be administered parenterally and which contain quinolonecarboxylic acids or derivatives thereof, of the general formula (I) 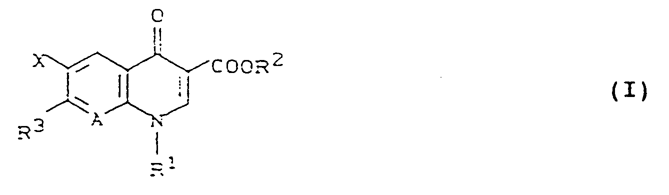 in which
R¹ represents methyl, ethyl, propyl, isopropyl, cyclopropyl, vinyl, 2-hydroxyethyl, 2-fluoroethyl, methoxy, amino, methylamino, dimethylamino, ethylamino, phenyl, 4-fluorophenyl or 2,4-difluorophenyl,
R² represents hydrogen, alkyl having 1 to 4 carbon atoms or (5-methyl-2-oxo-1,3-dioxol-4-yl)-methyl,
R³ represents methyl or a cyclic amino group, such as 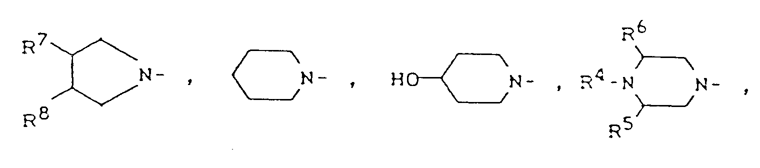 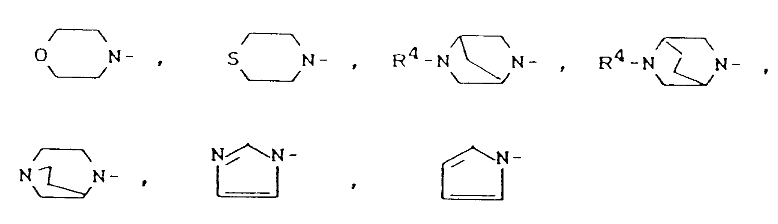 in which
R⁴ represents hydrogen, alkyl having 1 to 4 carbon atoms, 2-hydroxyethyl, allyl, propargyl, 2-oxopropyl, 3-oxobutyl, phenacyl, formyl, CFCl₂-S-CFCl₂-SO₂-, CH₃O-CO-S-, benzyl, 4-aminobenzyl 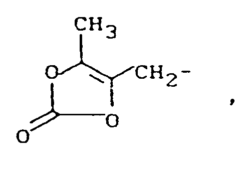
R⁵ represents hydrogen or methyl,
R⁶ represents hydrogen, alkyl having 1 to 4 carbon atoms, phenyl or benzyloxymethyl,
R⁷ represents hydrogen, amino, methylamino, ethylamino, aminomethyl, methylaminomethyl, ethylaminomethyl, dimethylaminomethyl, hydroxyl or hydroxymethyl,
R⁸ represents hydrogen, methyl, ethyl or chlorine,
X represents fluorine, chlorine or nitro, and
A represents N or C-R⁹, in which
R⁹ represents hydrogen, halogen, such as fluorine or chlorine, methyl or nitro, or alternatively, together with R¹, can form a bridge of the structure 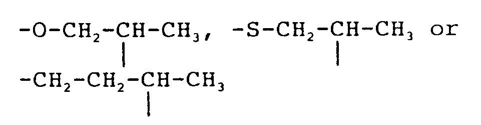
in dissolved form with no more than 1 to 10 ppm, preferably 1 to 5 ppm, relative to the major active compound component of the solution, of secondary components which have a tendency to precipitate, in particular after storage of the solution characterised in that an active compound of the formula (I) is dissolved using acid, the solution is allowed to stand, if appropriate with stirring, for 0.15 to 150 hours, preferably 0.25 to 110 hours, at temperatures from room temperature to the boiling point of the solution, if appropriate in the presence of activated charcoal, the solution is filtered, the quinolonecarboxylic acid is precipitated using basic reagents, and the precipitated quinolonecarboxylic acid is converted, using conventional auxiliaries, into an administration form which can be administered parenterally.

2. Process according to Claim 1, characterised in that the active compounds employed are those of the general formula (I) in which R³ represents the 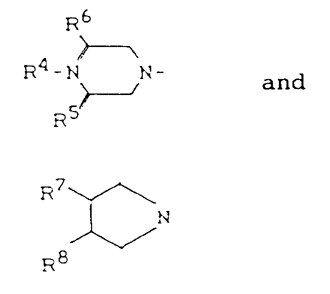 radicals
in which R⁴, R⁵, R⁶, R⁷ and R⁸ have the meaning specified in Claim 2.

3. Process according to Claims 1 and 2, characterised in that the active compounds employed are those of the general formula (I) in which X represents fluorine or chlorine and A represents CH, C-Cl, C-F or C-Br.

4. Process according to Claim 1, characterised in that the active compounds employed are those of the general formula (I) in which X represents fluorine and A represents CH, C-Cl or CF, and R³ denotes the 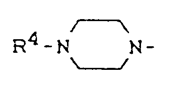 radical where R₄ = H, C₂H₅, CH₃ or -CH₂-CH₂-OH or the 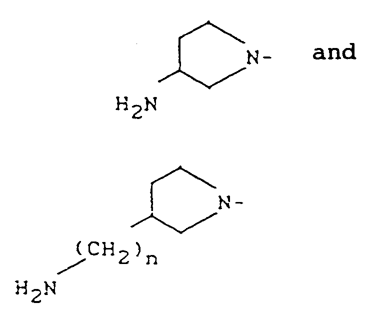 radicals where n = 1 to 4.

5. Process according to Claim 1, characterised in that the active compound used is ciprofloxacin.

6. Process according to Claim 5, characterised in that saturated ciprofloxacin solutions containing hydrochloric acid are allowed to stand for 0.15 to 150 hours at the particular temperature, and activated charcoal is employed.

7. Process according to Claim 6, characterised in that alkali metal hydroxides, alkaline-earth metal hydroxides, ammonia or organic bases are used for the precipitation.

## Revendications (Revendications pour l'(les) Etat(s) contractant(s) suivant(s): AT, BE, CH, DE, FR, GB, IT, LI, NL, SE)

1. Solutions d'acides quinolonecarboxyliques stables à l'entreposage, pour administration parentérale, caractérisées en ce qu'elles contiennent des acides quinolonecarboxyliques ou leurs dérivés de formule générale (I) 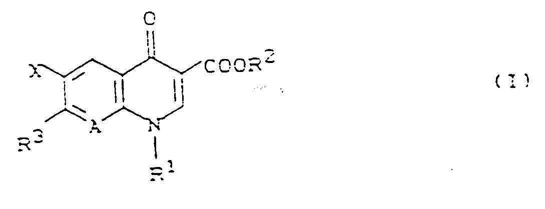 dans laquelle
R¹ signifie un groupe méthyle, éthyle, propyle, isopropyle, cyclopropyle, vinyle, 2-hydroxyéthyle, 2-fluoréthyle, méthoxy, amino, méthylamino, diméthylamino, éthylamino, phényle, 4-fluorophényle, 2,4-difluorophényle,
R² signifie l'hydrogène, un groupe alkyle avec 1 à 4 atomes de carbone, un groupe (5-méthyl-2-oxo-1,3-dioxol-4-yl)-méthyle,
R³ signifie méthyle ou un groupe amino cyclique comme 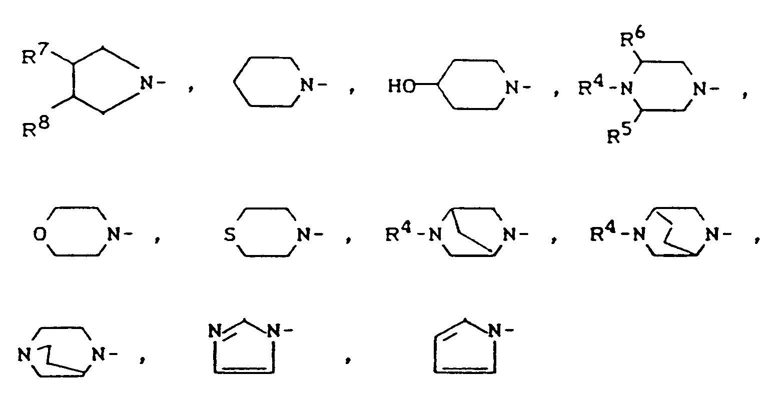 où
R⁴ signifie l'hydrogène, un groupe alkyle avec 1 à 4 atomes de carbone, un groupe 2-hydroxyéthyle, allyle, propargyle, 2-oxopropyle, 3-oxobutyle, phénacyle, formyle, CFCl₂-S-, CFCl₂-SO₂-, CH₃O-CO-S, benzyle, 4-aminobenzyle, 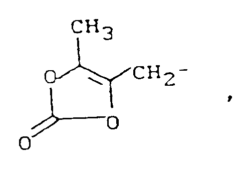
R⁵ signifie l'hydrogène, un groupe méthyle,
R⁶ signifie l'hydrogène, un groupe alkyle avec 1 à 4 atomes de carbone, un groupe phényle, un groupe benzyloxyméthyle,
R⁷ signifie l'hydrogène, un groupe amino, méthylamino, éthylamino, aminométhyle, méthylaminométhyle, éthylaminométhyle, diméthylaminométhyle, hydroxy, hydroxyméthyle,
R⁸ signifie l'hydrogène, un groupe méthyle, éthyle ou le chlore,
X signifie le fluor, le chlore ou un groupe nitro, et
A signifie N ou C-R⁹, dans lequel R⁹ signifie l'hydrogène, un halogène comme le fluor ou le chlore, un groupe méthyle ou nitro, ou peut former avec R¹ un pont de structure 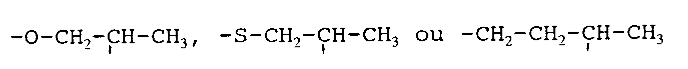
sous forme dissoute avec pas plus que 1 à 10 ppm, de préférence avec 1 à 5 ppm, par rapport au composant principal actif de la solution, de composants secondaires ayant tendance à former des précipités en particulier lors de l'entreposage de la solution.

2. Procédé pour fabriquer des solutions pour administration parentérale selon la revendication 1, caractérisé en ce que l'on dissout une substance de formule I avec de l'acide, après quoi on laisse reposer la solution à des températures pouvant aller de la température ambiante au point d'ébullition du solvant éventuellement en présence de charbon actif durant 0,15 à 150, de préférence 0,25 à 110 heures, on laisse reposer éventuellement sous agitation, on filtre la solution, on précipite l'acide quinolonecarboxylique avec des réactifs basiques et on met l'acide quinolonecarboxylique précipité sous une forme applicable par voie parentérale avec les adjuvants usuels.

3. Procédé selon la revendication 2, caractérisé en ce que l'on utilise comme substances actives des substances de formule générale (I), dans laquelle R₃ signifie les restes 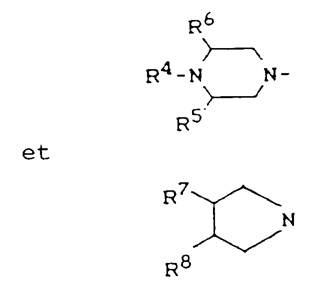 où R⁴, R⁵, R⁶, R⁷ et R⁸ ont les significations décrites dans la revendication 1.

4. Procédé selon la revendication 3, caractérisé en ce que l'on utilise comme substances actives des substances répondant à la formule générale I, dans laquelle X signifie le fluor ou le chlore et A signifie CH, C-Cl, C-F ou C-Br.

5. Procédé selon la revendication 4, caractérisé en ce que l'on utilise comme substances actives des substances répondant à la formule générale I, dans lesquelles R³ signifie le reste 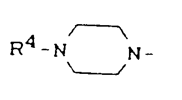 avec R₄ = H, C₂H₅, CH₃ ou -CH₂-CH₂-OH ou les restes 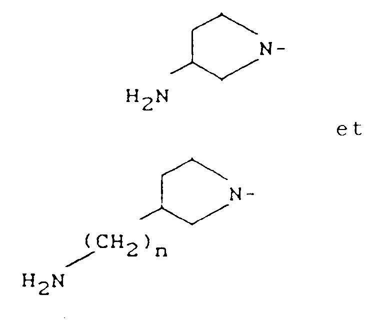 avec n = 1 à 4.

6. Procédé selon la revendication 2, caractérisé en ce que l'on emploie comme substance active la ciprofloxacine.

7. Procédé selon la revendication 6, caractérisé en ce qu'on laisse reposer des solutions de ciprofloxacine saturées dans l'acide chlorhydrique à température ambiante durant 0,15 à 150 heures et qu'on utilise du charbon actif.

8. Procédé selon la revendication 6, caractérisé en ce qu'on utilise des hydroxydes de métaux alcalins et alcalinoterreux, de l'ammoniac ou des bases organiques pour la précipitation.

9. Solutions ou substances actives fabriquées selon le procédé de la revendication 2, pour l'utilisation dans un procédé pour le traitement thérapeutique du corps humain ou d'animaux.

## Revendications (Revendications pour l'(les) Etat(s) contractant(s) suivant(s): GR, ES)

1. Procédé pour fabriquer des solutions pour administration parentérale, stables à l'entreposage, contenant des acides quinolonecarboxyliques ou leurs dérivés de formule générale (I) 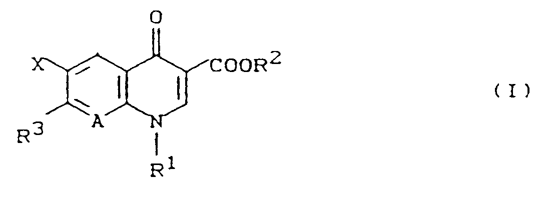 dans laquelle
R¹ signifie un groupe méthyle, éthyle, propyle, isopropyle, cyclopropyle, vinyle, 2-hydroxyéthyle, 2-fluoréthyle, méthoxy, amino, méthylamino, diméthylamino, éthylamino, phényle, 4-fluorophényle, 2,4-difluorophényle,
R² signifie l'hydrogène, un groupe alkyle avec 1 à 4 atomes de carbone, un groupe (5-méthyl-2-oxo-1,3-dioxol-4-yl)-méthyle,
R³ signifie méthyle ou un groupe amino cyclique comme 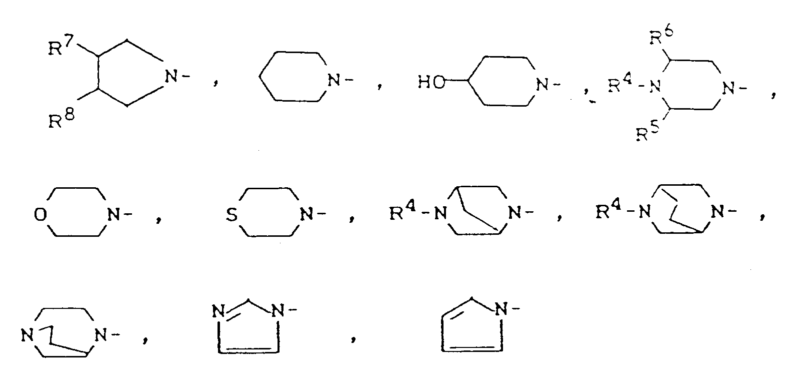 où
R⁴ signifie l'hydrogène, un groupe alkyle avec 1 à 4 atomes de carbone, un groupe 2-hydroxyéthyle, allyle, propargyle, 2-oxopropyle, 3-oxobutyle, phénacyle, formyle, CFCl₂-S-, CFCl₂-SO₂-, CH₃O-CO-S, benzyle, 4-aminobenzyle, 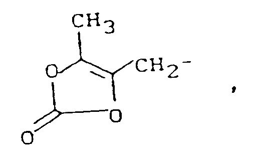
R⁵ signifie l'hydrogène, un groupe méthyle,
R⁶ signifie l'hydrogène, un groupe alkyle avec 1 à 4 atomes de carbone, un groupe phényle, un groupe benzyloxyméthyle,
R⁷ signifie l' hydrogène, un groupe amino, méthylamino, éthylamino, aminométhyle, méthylaminométhyle, éthylaminométhyle, diméthylaminométhyle, hydroxy, hydroxyméthyle,
R⁸ signifie l/hydrogène, un groupe méthyle, éthyle ou le chlore,
X signifie le fluor, le chlore ou un groupe nitro, et A signifie N ou C-R⁹, dans lequel R⁹ signifie l'hydrogène, un halogène comme le fluor ou le chlore, un groupe méthyle ou nitro, ou peut former ensemble avec R¹ un pont de structure 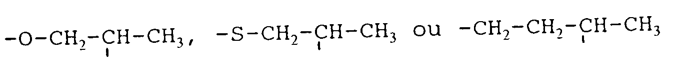
sous forme dissoute avec pas plus que 1 à 10 ppm, de préférence avec 1 à 5 ppm, par rapport au composant principal actif de la solution, de composants secondaires, en particulier ayant tendance à former des précipités après l'entreposage de la solution, procédé caractérisé en ce que l'on dissout une substance active de formule (I) avec de l'acide, on laisse reposer la solution à des températures pouvant aller de la température ambiante à la température du point d'ébullition de la solution, éventuellement en présence de charbon actif, durant 0,15 à 150 heures, de préférence 0,25 à 110 heures, éventuellement sous agitation, on filtre la solution, on précipite l'acide quinolonecarboxylique avec des réactifs basiques et on met l'acide quinolonecarboxylique sous une forme pouvant être administrée par voie parentérale avec les adjuvants usuels.

2. Procédé selon la revendication 1, caractérisé en ce qu'on utilise comme substance active des substances répondant à la formule (I), dans lesquelles R³ siqnifie les restes 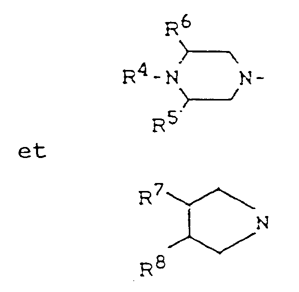 où R⁴, R⁵, R⁶, R⁷ et R⁸ possèdent la signification décrite dans la revendication 1.

3. Procédé selon les revendications 1 et 2, caractérisé en ce qu'on utilise comme substances actives des substances répondant à la formule générale (I) dans laquelle X signifie le fluor ou le chlore et A signifie CH, C-Cl, C-F ou C-Br.

4. Procédé selon la revendication 1, caractérisé en ce que l'on utilise comme substances actives des substances répondant à la formule générale (I) dans laquelle X signifie le fluor et A signifie CH, C-Cl ou CF et R³ signifie le reste 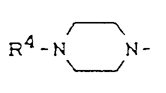 où R⁴ = H, C₂H₅, CH₃ ou -CH₂-CH₂-OH ou les restes 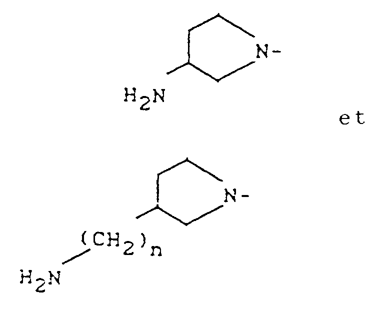 avec n = 1 à 4.

5. Procédé selon la revendication 1, caractérisé en ce que l'on emploie la ciprofloxacine comme substance active.

6. Procédé selon la revendication 5, caractérisé en ce qu'on laisse reposer à température des solutions de ciprofloxacine dans l'acide chlorhydrique durant 0,15 à 150 heures et qu'on utilise du charbon actif.

7. Procédé selon la revendication 6, caractérisé en ce qu'on utilise pour la précipitation des hydroxydes de métaux alcalins ou alcalinoterreux, de l'ammoniac ou des bases organiques.
